## Europäisches Patentamt

## European Patent Office

## Office européen des brevets

(19)

(11) Veröffentlichungsnummer: **0 224 193**
**B1**

(12)

# EUROPÄISCHE PATENTSCHRIFT

(45) Veröffentlichungstag der Patentschrift:
12.09.90

(51) Int. Cl.⁵: **A61N 2/08**

(21) Anmeldenummer: 86116043.0

(22) Anmeldetag: 20.11.86

(54) Vorrichtung zur Pflege und Behandlung der menschlichen Kopfhaut.

(30) Priorität: 27.11.85  CH 5064/85

(43) Veröffentlichungstag der Anmeldung:
03.06.87 Patentblatt 87/23

(45) Bekanntmachung des Hinweises auf die Patenterteilung:
12.09.90 Patentblatt 90/37

(84) Benannte Vertragsstaaten:
AT DE ES FR GB IT

(56) Entgegenhaltungen:
EP-A- 0 036 022
EP-A- 0 122 293
DE-A- 2 416 401
DE-A- 2 806 088
DE-A- 3 314 497
DE-A- 3 413 150
DE-C- 642 076

SOVIET INVENTIONS ILLUSTRATED, Sektion P, O,
Woche E34, 6. Oktober 1982, DERWENT PUBLICATIONS
LTD. London, P34

(73) Patentinhaber: Bodywell AG, Zürichstrasse 31,
8700 Küsnacht ZH(CH)

(72) Erfinder: Schaerer, Emil A., Zürichstrasse 31,
CH-8700 Küsnacht(CH)

(74) Vertreter: Petschner, Goetz, Patentanwaltsbüro G.
Petschner Seidengasse 18, CH-8001 Zürich(CH)

## Beschreibung

Die vorliegende Erfindung betrifft eine Vorrichtung zur Pflege und Behandlung der menschlichen Kopfhaut, wie aus DE-A 3 314 497 bekannt.

Es ist bekannt, dass Haarschwund bzw. Haarausfall bei Menschen vielfach auf Ernährungsstörung der Haarwurzel, bedingt durch Zusammenpressen der Blutgefässe infolge von Zugwirkung der die Kopfhaut spannenden Muskulatur, zurückzuführen ist. (Brockhaus Enzyklopädie 1969). Um hier eine entgegenwirkende bessere Durchblutung der Haarwurzel zu erreichen, sind kräftige Kopfmassagen üblich, die aber in der Regel unangenehm, nicht immer anwendbar und u.U. schädlich für das Oberhaar sind.

Andrerseits ist es seit Urzeiten bekannt, dass die Magnetenergie von auf die Haut aufgelegten Biomagneten eine starke Durchblutung der benachbarten Blutgefässe zur Folge hat; und zudem wird angenommen, dass die Magnetenergie auf Kalkablagerungen an den organischen Gefässwänden hindernd und abbauend Einfluss nimmt (Dr.sc.tech.ETH Walter O. Stark, "Magnetismus in der Therapie").

Es ist nun Aufgabe der vorliegenden Erfindung, eine Vorrichtung zur Pflege und Behandlung der Kopfhaut zu schaffen, welche gestattet, die "sanfte" Massage der Magnetenergie zur Erzielung einer höheren Durchblutung der Kopfhaut auf bequeme Art für die Anwender zu nutzen.

Dies wird erfindungsgemäss zunächst erreicht durch die Vorrichtung gemäß Anspruch 1.

Diese Massnahmen gestatten, die Biomagnete ganz präzise auf den vorbestimmten Stellen des Kopfes zu plazieren und geben dem Anwender Gelegenheit, sich der Wirkung der Biomagnete längere Zeit, beispielsweise über Nacht auszusetzen, ohne dabei besondere Vorkehrungen für die ortsfeste Fixierung der Biomagnete treffen zu müssen, da die Kopfbedeckung den Kopf in der Regel unverrückbar umschliesst.

Hierbei können verschiedene Verfahren zur Anwendung kommen, etwa ist es möglich, dass an jeder vorgegebenen Stelle zwei gegenpolig aufeinanderliegende, die Kopfbedeckung zwischen sich einspannende Permanentmagnetträger angeordnet sind, oder dass an jeder vorgegebenen Stelle bei aufgesetzter Kopfbedeckung auf diese aussenseitig ein Markierungsplättchen oder -pflaster sowie bei abgenommener Kopfbedeckung auf diese innenseitig auf der Gegenseite des Markierungsplättchens ein Permanentmagnetträger aufbringbar ist.

Eine optimale Wirkung wird dabei erreicht, wenn die dauermagnetischen Teilchen eine magnetische Flussdichte von $8.10^{-2}$ bis $9.10^{-2}$ Tes/a 800 bis 900 Gauss aufweisen.

Eine einfache Handhabung wird zudem gewährleistet, wenn die Markierungsplättchen bzw. -Pflaster und die Permanentmagnetträger selbsthaftend sind.

Es wurde zudem gefunden, dass die Wirkung optimal ist, wenn die dauermagnetischen Teilchen Barium- oder Strontiumferrite sind.

Ferner können Wirksamkeit und Einfluss auf das Haarwachstum erhöht werden, wenn den dauermagnetischen Teilchen aus Barium- oder Strontiumferriten Zinksulfatteilchen oder -lösungen zugegeben sind.

Beispielsweise Ausführungsformen des Erfindungsgegenstandes sind nachfolgend anhand der Zeichnung näher erläutert. Es zeigen:

Fig. 1 in schaubildartiger Darstellung die Vorrichtung zur Pflege und Behandlung der menschlichen Kopfhaut, in einer ersten Vorbereitungsphase;

Fig. 2 in schaubildartiger Darstellung die Vorrichtung gemäss Fig. 1 in einer weiteren Vorbereitungsphase;

Fig. 3 in Draufsicht einen Permanentmagnetträger der Anordnung gemäss den Fig. 1 und 2; und

Fig. 4 im Querschnitt eine Anordnung von Permanentmagnetträgern in Selbsthaftung an der Kopfbedeckung.

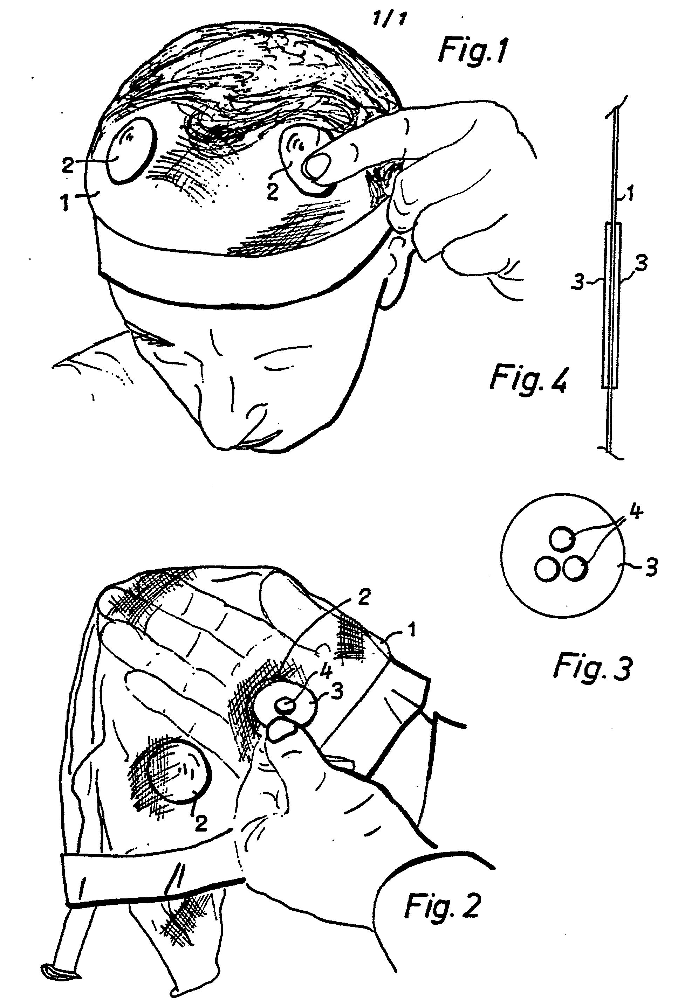

Aus den Fig. 1 und 2 ist die erfindungsgemässe Vorrichtung zur Pflege und Behandlung der menschlichen Kopfhaut sowie die Phasen deren individueller Anpassung entnehmbar.

Vorzugsweise wird eine transparente Haube oder ein übliches Haarnetz 1 verwendet, das zunächst in richtigem Sitz auf den Kopf gesetzt wird (Fig. 1). Bei aufgesetztem Haarnetz 1 werden dann auf diesem an den gewünschten Stellen, an denen die Kopfhaut dem Einfluss der Biomagnete ausgesetzt werden soll, aufklebbare Markierungsplättchen oder -pflaster 2 aufgeklebt (Fig. 1).

Anschliessend wird das Haarnetz 1 wieder abgenommen und auf dieses innenseitig auf der Gegenseite der Markierungsplättchen 2 folien- oder plättchen- oder pflasterförmige Permanentmagnetträger 3 aufgeklebt (Fig. 2).

Diese Permanentmagnetträger 3 tragen hier je mindestens einen Permanentmagnetkern 4 und zwar derart, dass dieser bzw. diese dann bei wieder aufgesetztem Haarnetz 1 mit der Kopfhaut des Benützers genau an den vorher bestimmten Stellen in Berührung stehen. Hierbei wird in der Regel die negative (weisse) Polseite vom Träger 3 bzw. Haarnetz 1 abragen.

Vorteilhaft ist es, wenn jeder Permanentmagnetträger 3 drei, mit vorgegebenem Abstand voneinander und unterschiedlicher Nord-Süd-Polung aufgebrachte Permanentmagnetkerne 4 umfasst, wie das in Fig. 3 näher veranschaulicht ist.

Die Markierungsplättchen und die Permanentmagnetträger weisen vorzugsweise einen Durchmesser von 5 cm und die Permanentmagnetkerne einen Durchmesser von etwa 1 cm auf.

Die Flussdichte der Biomagnete 4 sollte ca. 800 bis 900 Gauss betragen.

Ferner sind die Markierungsplättchen bzw. Pflaster 2 und die Permanentmagnetträger 3 selbsthaftend oder selbstklebend ausgerüstet.

Aus dem Vorbeschriebenen ergibt sich nun eine Vorrichtung zur Pflege und Behandlung der menschlichen Kopfhaut, welche dem Benützer eine leichte, bequeme und individuell anpassbare Handhabung gestattet.

Dabei sind im Rahmen der Erfindung natürlich eine Reihe von Modifikationen denkbar. Beispielsweise kann das Haarnetz, wie erwähnt, durch eine Haube aus irgend einem durchsichtigen Material ersetzt sein. Ferner kann jeder Permanentträger mit auch zwei oder mehr als drei Biomagnete bestückt sein mit gleicher oder ungleicher Polung bzw. mit gleicher oder ungleicher Flussdichte.

Der Permanentmagnetträger kann weiter auch eine bekannte biegsame magnetische Folie für therapeutische Zwecke sein, in der die dauermagnetischen Teilchen eingebettet sind. Diese dauermagnetischen Teilchen der Folie oder der Kerne sind vorzugsweise Barium- oder Strontiumferrite, denen bekannte Haarwuchsmittel beigegeben sein können, wie Zinksulfatteilchen oder -lösungen.

Sind die Permanentmagnetträger solche vorgenannten biegsamen magnetischen Folien, können auch, wie in Fig. 4 ver anschaulicht, an jeder vorgebenen Stelle zwei gegenpolig aufeinanderliegende, die Kopfbedeckung I zwischen sich einspannende Permanentmagnetträger 3 angeordnet sein. Entsprechende Markierungen auf den Folien und eventuell eine rutschfeste Beschichtung auf der mit der Kopfhaut in Berührung zu bringenden Seite der Folie können deren Anbringung erleichtern.

## Patentansprüche

1. Vorrichtung zur Pflege und Behandlung der menschlichen Kopfhaut, mit einer Magnetmittel tragenden Kopfbedeckung, dadurch gekennzeichnet, dass die Kopfbedeckung ein Haarnetz (1) oder eine transparente Haube ist, an welcher an vorgegebenen Stellen die Magnetmittel in Form von folien- oder plättchen- oder pflasterförmigen, dauermagnetische Teilchen aufweisenden Permanentmagnetträgern (3) anbringbar sind, derart, dass jeweils zwei gegenpolig aufeinanderliegende, die Kopfbedeckung (1) zwischen sich einspannende Permanentmagnetträger (3) angeordnet sind, wobei an jeder vorgegebenen Stelle bei aufgesetzter Kopfbedeckung auf diese aussenseitig ein Markierungsplättchen (2) oder -pflaster sowie bei abgenommener Kopfbedeckung (1) auf diese innenseitig auf der Gegenseite des Markierungsplättchens (2) ein Permanentmagnetträger (3) aufbringbar ist.

2. Vorrichtung nach Anspruch 1, dadurch gekennzeichnet, dass die dauermagnetischen Teilchen eine magnetische Flussdichte von $8 \cdot 10^{-2}$ bis $9 \cdot 10^{-2}$ Tesla (800 bis 900 Gauss) aufweisen.

3. Vorrichtung nach Anspruch 1, dadurch gekennzeichnet, dass die Markierungsplättchen bzw. -pflaster (2) und die Permanentmagnetträger (3) selbsthaftend sind.

4. Vorrichtung nach Anspruch 1, dadurch gekennzeichnet, dass die dauermagnetischen Teilchen Barium- oder Strontiumferrite sind.

5. Vorrichtung nach Anspruch 4, dadurch gekennzeichnet, dass den dauermagnetischen Teilchen aus Barium- oder Strontiumferriten Zinksulfatteilchen oder -lösungen zugegeben sind.

## Revendications

1. Dispositif pour soins et traitement du cuir chevelu humain, comportant une couverture de la tête portant un moyen magnétique, caractérisé en ce que la couverture de la tête est une résille de cheveux (1) ou une calotte transparente, sur laquelle, à des endroits prédéterminés, les moyens magnétiques peuvent être rapportés sous la forme de supports d'aimants permanents (3) présentant des particules d'aimants permanents sous la forme de feuilles, de plaquettes ou de pavés, de telle façon que l'on dispose chaque fois deux supports d'aimants permanents (3) placés en opposition de pôle l'un par rapport à l'autre et mettant en tension entre eux la couverture 1 de la tête, étant entendu qu'à chacun des endroits prédéterminés, lorsqu'on a mis en place la couverture de la tête, on peut disposer sur celle-ci, sur sa face externe, une plaquette, ou un pavé, de marquage (2) ainsi qu'après enlèvement de la couverture de la tête (1), sur sa face interne, en face de la plaquette de marquage (2), on peut disposer un support d'aimant permanent (3).

2. Dispositif suivant la revendication 1, caractérisé en ce que les éléments d'aimants permanents présentent une densité de flux de $8 \cdot 10^{-2}$ à $9 \cdot 10^{-2}$ tesla (800 à 900 Gauss).

3. Dispositif suivant la revendication 1, caractérisé en ce que les plaquettes, ou pavés, de marquage (2) et les supports d'aimants permanents (3) sont auto-adhésifs.

4. Dispositif suivant la revendication 1, caractérisé en ce que les particules d'aimants permanents sont des ferrites de baryum ou de strontium.

5: Dispositif suivant la revendication 4, caractérisé en ce qu'aux particules d'aimants permanents de ferrites de baryum ou de strontium sont ajoutées des particules ou des solutions de sulfate de zinc.

## Claims

1. Device for the care and therapy of the human scalp, wherein the head covering is a hair net (1) or a transparent hood, on to which the magnet means, in the form of foil-shaped or small plate-shaped or plaster-shaped permanent magnet bearers (3), having permanent magnetic particles, are attachable in such a manner that in each case two superimposed, permanent magnet bearers (3) of opposite polarity are arranged, gripping the head covering (1) between each other; and, at each preselected place of the head covering (1), when this is worn, a small marking plate (3) or plaster is attachable to the outside thereof, as well as, when the head covering is removed, a permanent magnet bearer (3) being attachable to the inside thereof on the opposite side of the small marking plate (2).

2. Device as defined in claim 1, wherein the permanent magnetic particles possess a magnetic flux density of $8 \cdot 10^{-2}$ to $9 \cdot 10^{-2}$ Tesla (800 to 900 gauss).

3. Device as defined in claim 1, wherein the small marking plates or plasters (2) and the permanent-magnetic bearers (3) are self-adhesive.

4. Device as defined in claim 1, wherein the permanent-magnetic particles are barium ferrite or strontium ferrite.

5. Device as defined in claim 4, wherein particles or solutions of zinc sulphate are added to the permanent magnetic particles of barium ferrite or strontium ferrite.

Fig.1

Fig. 4

Fig. 2

Fig.3